Europäisches Patentamt

European Patent Office

Office européen des brevets

(19)

(11) Veröffentlichungsnummer: **0 021 010**
**B1**

(12) **EUROPÄISCHE PATENTSCHRIFT**

(45) Veröffentlichungstag der Patentschrift:
12.05.82

(21) Anmeldenummer: 80102714.5

(22) Anmeldetag: 16.05.80

(51) Int. Cl.³: **C 07 C 69/34,** C 07 C 69/44,
C 07 C 67/38 // C07C69/533

(54) Verfahren zur Herstellung von Butandicarbonsäureestern.

(30) Priorität: 20.06.79 DE 2924785

(43) Veröffentlichungstag der Anmeldung:
07.01.81 Patentblatt 81/1

(45) Bekanntmachung des Hinweises auf die Patenterteilung:
12.05.82 Patentblatt 82/19

(84) Benannte Vertragsstaaten:
BE CH DE FR GB IT LI NL

(56) Entgegenhaltungen:
keine

(73) Patentinhaber: BASF Aktiengesellschaft,
Carl-Bosch-Strasse 38, D-6700 Ludwigshafen (DE)

(72) Erfinder: Kummer, Rudolf, Dr., Kreuzstrasse 6,
D-6710 Frankenthal 5 (DE)
Erfinder: Schneider, Heinz-Walter, Dr., Bruesseler
Ring 43, D-6700 Ludwigshafen (DE)
Erfinder: Taglieber, Volker, Dr., Beuthener Strasse 7,
D-6700 Ludwigshafen (DE)
Erfinder: Weiss, Franz-Josef, Dr., Schlehdornweg 69,
D-6940 Weinheim (DE)

Verfahren zur Herstellung von Butandicarbonsäureestern

Gegenstand der Erfindung ist ein Verfahren zur Herstellung von Butandicarbonsäureestern, bei dem man Butadien oder Butadien enthaltende Kohlenwasserstoffgemische mit Kohlenmonoxid und niederen Alkoholen in Gegenwart von tertiären Stickstoffbasen und Kobaltcarbonyl bei Temperaturen von 80 bis 150°C unter erhöhtem Druck umsetzt und den so erhaltenen Pentensäureester mit Kohlenmonoxid und niederen Alkoholen bei Temperaturen von 140 bis 200°C unter erhöhtem Druck weiter zu Butandicarbonsäureestern umsetzt.

Aus Bull. Chem. Soc., Japan, 46, S. 524 ff. (1973), ist ein zweistufiges Verfahren zur Herstellung von Adipinsäureestern aus Butadien bekannt, wobei man Butadien zunächst mit Kohlenmonoxid und Alkanolen in Gegenwart von Kobaltcarbonyl und Stickstoffbasen, wie Pyridin oder Isochinolin, umsetzt, ohne den Katalysator zu entfernen, in einer darauffolgenden Stufe den entstandenen Pentensäureester mit Kohlenmonoxid und Alkanolen weiter zu Adipinsäure umsetzt. Bei der technischen Durchführung eines solchen Verfahrens ist es jedoch erforderlich, den Katalysator wiederzugewinnen und zurückzuführen. So wurde in dem aus der US-PS 3 778 466 bekanntgewordenen Verfahren der den Katalysator enthaltende Rückstand nach Abdestillieren der Wertprodukte wieder für die Carbonylierung verwendet. Es hat sich jedoch gezeigt, daß beispielsweise nach 4maliger Verwendung die Wirksamkeit des Katalysators erheblich nachläßt. Dies ist darauf zurückzuführen, daß einerseits durch die Destillation der Katalysator geschädigt wird, da Kobaltcarbonylkomplexe thermisch nicht stabil sind und andererseits bei der Carbonylierung Nebenprodukte entstehen, die die Carbonylierung beeinflussen und deshalb fortlaufend ausgeschleust werden müssen. Es wurde auch schon versucht, nach der Carbonylierung die Wertprodukte durch Extraktion vom katalysatorhaltigen Rückstand abzutrennen. So wird in der DE-OS 2 159 139 ein Verfahren beschrieben, bei dem man das Methanol enthaltende Carbonylierungsgemisch mit Kohlenwasserstoffen extrahiert. Hierbei gelingt es zwar ohne Schädigung des Katalysators die Wertprodukte abzutrennen und methanolische Katalysator enthaltende Lösung in die Carbonylierung zurückzuführen. Diese extraktive Trennung ist jedoch nicht geeignet, um Nebenprodukte wie polymere Butadiene, die bei der Carbonylierung entstehen, auszuschleusen. Sie reichern sich deshalb bei mehrmaliger Wiederverwendung der Katalysatorlösung fortlaufend an und führen zu Störungen in der Carbonylierung. Entsprechend der DE-OS 2 741 511 verwendet man Kohlenwasserstoffe, Ketone oder Äther als Extraktionsmittel für den in eine wäßrige Lösung überführten Katalysatorstrom. Abgesehen davon, daß eine Extraktionskolonne mit vielen Trennstufen erforderlich ist, gelingt es nicht, die Nebenprodukte in dem erforderlichen, für einen ungestörten Reaktionsablauf notwendigen Ausmaß zu entfernen.

Es war deshalb die technische Aufgabe gestellt, die zurückzuführende wäßrige Kobaltsalzlösung so zu behandeln, daß keine Störungen im Ablauf der Carbonylierung eintreten, und zudem die Extraktion einfacher gestaltet wird.

Diese Aufgabe wird gelöst, in einem Verfahren zur Herstellung von Butandicarbonsäureestern, bei dem man Butadien oder Butadien enthaltende Kohlenwasserstoffgemische mit Kohlenmonoxid und $C_1$- bis $C_4$-Alkanolen in Gegenwart von tertiären Stickstoffbasen und Kobaltcarbonylen bei 80 bis 150°C unter erhöhtem Druck umsetzt, ohne den Katalysator abzutrennen, dann den erhaltenen Pentensäureester mit Kohlenmonoxid und $C_1$- bis $C_4$-Alkanolen bei Temperaturen von 140 bis 200°C unter erhöhtem Druck zu Butandicarbonsäureestern umsetzt, wobei man

a) eine wäßrige Kobaltsalzlösung bei Temperaturen von 50 bis 200°C und unter Drücken von 50 bis 500 bar mit überschüssigem Kohlenmonoxid und Wasserstoff in Gegenwart von Aktivkohle, die Kobaltcarbonyl beladen ist, behandelt;

b) die erhaltene wäßrige Lösung von Kobaltcarbonylwasserstoff mit Butadien oder Butadien enthaltenden Kohlenwasserstoffgemischen extrahiert und die wäßrige Phase abtrennt;

c) das Kobaltcarbonylhydrid, Kobaltcarbonyl und Butenylkobalttricarbonyl enthaltende Butadien oder Butadienkohlenwasserstoffgemisch mit Kohlenmonoxid und $C_1$- und $C_4$-Alkanolen im Überschuß in Gegenwart von 0,5 bis 2 Mol tertiäre Stickstoffbasen je Mol Butadien mit einem $pK_a$-Wert von 3 bis 11 bei Temperaturen von 80 bis 150°C unter Drücken von 300 bis 2000 bar umsetzt;

d) aus dem erhaltenen Reaktionsgemisch die darin enthaltenen tertiären Stickstoffbasen bis auf 0,1 bis 0,3 Mol je Mol Pentensäureester sowie überschüssige Kohlenwasserstoffe abtrennt, den im Reaktionsgemisch verbleibenden Pentenester mit Kohlenmonoxid und $C_1$- bis $C_4$-Alkanolen im Überschuß bei Temperaturen von 140 bis 200°C und unter Drücken von 100 bis 400 bar in Gegenwart der im Reaktionsgemisch vorhandenen Menge an Kobaltcarbonyl und tertiären Stickstoffbasen umsetzt;

e) das Kobaltcarbonyl, Alkanole, Stickstoffbasen, Butandicarbonsäureester und Nebenprodukte sowie unumgesetzten Pentenester enthaltende Reaktionsgemisch mit Oxidationsmitteln in Gegenwart der wäßrigsauren Lösung, die in Stufe b) abgetrennt wurde, behandelt und das Gemisch in eine organische Phase, aus der durch Destillation Butandicarbonsäureester gewonnen werden, und eine wäßrige Phase trennt, und

f) die wäßrige Phase mit Lösungsmitteln extrahiert, die mit Wasser nicht mischbar sind, die Phasen trennt und die so erhaltene wäßrige Phase nach Abdestillieren von Alkanolen und tertiären Stickstoffbasen in die Stufe a) zurückführt,

dadurch gekennzeichnet, daß man als Extraktionsmittel in der Stufe f) $C_1$- bis $C_5$-Alkan- und/oder Alkencarbonsäure $C_1$- bis $C_4$-Alkylester verwendet.

Das neue Verfahren hat den Vorteil, daß man weniger Extraktionsstufen benötigt und erheblich geringere Mengen an Extraktionsmitteln aufwenden muß. Darüber hinaus hat das neue Verfahren den Vorteil, daß insbesondere den Katalysator schädigende Nebenprodukte noch vollständiger entfernt werden als bisher. Schließlich hat das neue Verfahren den Vorteil, daß bei Verwendung von Pentan- oder Pentensäurealkylestern Produkte, die von sich aus während des Herstellungsvorganges anfallen, als Extraktionsmittel verwendet werden und keine synthesefremden Extraktionsmittel eingesetzt werden müssen.

In einer ersten Stufe (Stufe a) werden wäßrige Kobaltsalzlösungen mit Kohlenmonoxid und Wasserstoff im Überschuß bei Temperaturen von 50 bis 200°C und unter Drücken von 50 bis 500 bar in Gegenwart von Aktivkohle, die mit Kobaltcarbonyl beladen ist, behandelt. Bevorzugt verwendet man als Kobaltsalze fettsaure Salze, die wasserlöslich sind, insbesondere Formiate, Acetate, Propionate oder Butyrate. Besonders bewährt haben sich Kobaltformiat und Acetat. Zweckmäßig geht man von Lösungen aus, die 0,5 bis 5 Gew.-% Kobalt, berechnet als Metall, insbesondere 1 bis 3 Gew.-% Kobalt in Form der genannten Salze enthalten. Im allgemeinen enthält das genannte Gasgemisch Kohlenmonoxid und Wasserstoff im Volumenverhältnis 4 : 1 bis 1 : 2, insbesondere im Volumenverhältnis 2 : 1 bis 1 : 1. Besonders bewährt hat sich ein annähernd äquimolekulares Gemisch aus Kohlenmonoxid und Wasserstoff. Vorteilhaft verwendet man das Gemisch aus Kohlenmonoxid und Wasserstoff in einem Überschuß, z. B. bis zu dem 5fachen der stöchiometrisch erforderlichen Menge. Vorteilhaft hält man Temperaturen von 100 bis 170°C und Drücke von 100 bis 400 bar ein.

Die Behandlung in der Stufe a) erfolgt in Gegenwart von Aktivkohle. Geeignete Aktivkohlearten sind z. B. Torfkohle, Tierkohle oder Zuckerkohle. Als besonders geeignet hat sich Torfkohle erwiesen. Die Aktivkohle ist zweckmäßig bis zu deren Sättigung mit Kobaltcarbonyl beladen. Dies wird im allgemeinen dadurch erreicht, daß man wäßrige Lösungen von Kobaltsalzen zusammen mit dem genannten Gasgemisch aus Kohlenmonoxid und Wasserstoff unter den angegebenen Reaktionsbedingungen über die Aktivkohle bis zu deren Sättigung leitet, d. h. bis man im Austrag Kobaltcarbonyl oder Kobaltcarbonylwasserstoff analytisch feststellt.

Im allgemeinen führt man die Behandlung in einer sogenannten Behandlungszone durch, die zweckmäßig ein Verhältnis von Länge zu Durchmesser von 5 bis 50 : 1 hat, in der die Aktivkohle in der Regel fest angeordnet ist. Vorteilhaft hält man eine Belastung von 1,5 bis 15 g Kobalt, berechnet als Metall in Form der genannten Salze pro Stunde je Kilogramm Aktivkohle ein.

Die so erhaltene Kobaltcarbonylwasserstoff nicht umgesetzte Kobaltsalze und freigesetzte Säure enthaltende wäßrige Lösung wird zweckmäßig zusammen mit dem nicht verbrauchten Gemisch aus Kohlenmonoxid und Wasserstoff vorteilhaft ohne Entspannen der zweiten Stufe (Stufe b) zugeführt. Dort wird Kobaltcarbonylwasserstoff mit Butadien oder Butadien enthaltenden Kohlenwasserstoffgemischen, die nachfolgend noch näher erläutert werden, extrahiert. Es ist möglich, die gesamte für die Carbonylierung erforderliche Butadienmenge zur Extraktion zu verwenden oder nur einen Teil derselben. Vorteilhaft verwendet man 50 bis 30 Mol Butadien pro Grammatom zu extrahierendes Kobalt. Die Extraktion wird im Gegenstrom oder Gleichstrom in Vorrichtungen durchgeführt, die in der Technik für die Extraktion eingeführt sind, beispielsweise Kolonnen oder statische Mischer. Während der Extraktion hält man Temperaturen von 20 bis 100°C und Drücke von 5 bis 300 bar ein. Das Gemisch wird anschließend in eine wäßrige und eine organische Phase getrennt. Führt man die Extraktion beispielsweise in einem mit Raschig-Ringen gefüllten Druckrohr aus, so tritt im oberen Teil gleichzeitig eine Trennung in eine organische und eine wäßrige Phase ein. Gleichzeitig wird das mitverwendete Gemisch aus Kohlenmonoxid und Wasserstoff als Gasphase abgetrennt. Der Kobaltgehalt der die Stufe b) verlassenden organischen Phase beträgt im allgemeinen von 1 bis 5 Gew.-%. Es wird angenommen, daß das Kobaltcarbonyl als Komplexverbindung mit Butadien, die in Wasser unlöslich ist, in der organischen Phase vorliegt.

Die organische Phase wird nun in der Stufe c) in Gegenwart von 0,5 bis 2 Mol tertiärer Stickstoffbasen je Mol Butadien mit einem $pK_a$-Wert von 3 bis 11 mit der Maßgabe, daß die tertiäre Stickstoffbase vorzugsweise niedriger sieden soll, als der jeweils herzustellende Pentensäureester mit $C_1$- bis $C_4$-Alkanolen im Überschuß bei Temperaturen von 80 bis 150°C und unter Drücken von 300 bis 2000 bar umgesetzt.

Falls für die Extraktion nicht die gesamte Menge an Butadien oder Butadien enthaltendes Kohlenwasserstoffgemisch, das für die Carbonylierung benötigt wird, verwendet wurde, setzt man die erforderliche Menge an Ausgangsstoffen in der Stufe c) zusätzlich zu. Es sei hier vermerkt, daß anstatt reinem Butadien vorteilhaft Butadien enthaltende Kohlenwasserstoffgemische verwendet werden können. Solche Kohlenwasserstoffgemische enthalten neben Butadien gesättigte Kohlenwasserstoffe mit 3 bis 5 Kohlenstoffatomen und einfach olefinisch ungesättigte Olefine mit 3 bis 5 Kohlenstoffatomen. In der Technik werden insbesondere $C_4$-Schnitte als Ausgangsgemische verwendet, die bei der Dehydrierung von Butan oder Buten oder als Nebenprodukte bei der Äthylengewinnung durch thermische Spaltung von Leichtbenzin oder höheren Kohlenwasserstoffschnitten anfallen. Solche Gemische enthalten in der Regel 40 bis 60 Gew.-% Butadien, 20 bis 35 Gew.-% Isobuten, 10 bis 25 Gew.-% Buten-1, 5 bis 15 Gew.-% Buten-2, 1 bis 10 Gew.-% Butane und 0,1 bis 3 Gew.-% Butine.

Geeignete tertiäre Stickstoffbasen sind vorzugsweise N-heterocyclische Verbindungen, wie Pyridin ($pK_a$ 5,3), Methylpyridine wie 3-Pikolin ($pK_a$ 6,0), Isochinolin ($pK_a$ 5,4), besondere technische Bedeutung hat Pyridin erlangt. Es hat sich besonders bewährt, 0,6 bis 1,5 Mol tertiäre Stickstoffbasen je Mol Butadien anzuwenden.

Geeignete $C_1$- bis $C_4$-Alkanole sind Methanol, Äthanol, Propanol, Butanol, Isobutanol, besonders bevorzugt wird Methanol verwendet.

Die Umsetzung führt man vorzugsweise bei Temperaturen von 120 bis 140°C und Drücken von 600 bis 1200 bar durch. Je Mol Butadien verwendet man in der Regel 0,01 bis 0,1 Grammatom Kobalt in Form der beschriebenen Carbonylkomplexe.

Das so erhaltene Reaktionsgemisch enthält neben nicht umgesetztem Butadien gegebenenfalls andere Kohlenwasserstoffe, tertiäre Stickstoffbasen, Kobaltcarbonylkomplexe, nicht umgesetzte Alkanole, die als Wertprodukte erzeugten Pentensäureester sowie Nebenprodukte wie Valeriansäureester, Vinylcyclohexen, Butenyl- und Butylketone sowie Polymerisate des Butadiens.

Aus dem so erhaltenen Reaktionsgemisch werden nach dem Entspannen die darin enthaltenen tertiären Stickstoffbasen bis auf 0,1 bis 0,3 Mol je Mol Pentensäureester sowie gegebenenfalls überschüssige Kohlenwasserstoffe abgetrennt (Stufe d). Die Abtrennung kann durch Destillation oder andere Trennverfahren, wie Extraktion, erfolgen. Vorteilhaft werden tertiäre Stickstoffbasen, gegebenenfalls überschüssige Kohlenwasserstoffe durch Destillation unter vermindertem Druck abgetrennt. Hierbei soll die Temperatur im Destillationssumpf 75°C nicht überschreiten, um eine Zersetzung des Kobaltkatalysators zu vermeiden. Je nach Wahl des mitverwendeten Alkanols destilliert gleichzeitig auch ein Teil oder das gesamte überschüssige Alkanol ab.

Der im Reaktionsgemisch verbleibende Pentensäureester wird mit Kohlenmonoxid und $C_1$- bis $C_4$-Alkanolen im Überschuß nach dem gegebenenfalls eine entsprechende Menge Alkanole erneut zugegeben wurde, bei Temperaturen von 140 bis 200°C und unter Drücken von 100 bis 400 bar in Gegenwart der im Reaktionsgemisch vorhandenen Menge an Kobaltkatalysator und tertiärer Stickstoffbase umgesetzt. Vorteilhaft hält man Temperaturen von 150 bis 180°C ein. Vorzugsweise beträgt die mitverwendete Menge an Alkanolen 1,5 bis 4 Mol je Mol Pentensäureester. Es hat sich ferner als vorteilhaft erwiesen, dem Kohlenmonoxid einige Volumprozent Wasserstoff, z. B. 1 bis 4 Volumprozent, zuzugeben.

Das Katalysator Butandicarbonsäureester, Alkanole, tertiäre Stickstoffbasen sowie Pentensäureester und Nebenprodukte enthaltende Reaktionsgemisch wird in der Stufe e) mit Oxidationsmitteln in wäßrigsaurem Medium behandelt. Als Oxidationsmittel sind insbesondere solche geeignet, die das Reaktionsgemisch nicht verunreinigen, beispielsweise Wasserstoffperoxid, Sauerstoff oder Sauerstoff enthaltende Gase. Besonders bevorzugt werden molekularen Sauerstoff enthaltende Gase, insbesondere Luft, verwendet. Das Oxidationsmittel wird mindestens in einer Menge von zwei Oxidationsäquivalenten je Mol Kobaltverbindung angewendet. Vorteilhaft wendet man jedoch ein Überschuß an, in der Praxis hat es sich als zweckmäßig erwiesen, 30 bis 300 Nl Luft je Kilogramm Reaktionsgemisch anzuwenden.

Im allgemeinen verwendet man die 0,1- bis 10fache, vorteilhaft 0,2- bis 1fache Gewichtsmenge Wasser, bezogen auf das Reaktionsgemisch. Der pH-Wert beträgt vorteilhaft zwischen 3 bis 6. Ein geeignetes saures Medium ist die in der Stufe b) anfallende wäßrige saure Lösung nach Abtrennung des Kobaltcarbonylwasserstoff enthaltenden Butadiens. Eine solche Lösung enthält z. B., falls man vom Kobaltacetat ausgeht, neben nicht umgesetztem Kobaltacetat Essigsäure. Falls es erforderlich ist, kann zusätzlich eine geeignete Fettsäure zugegeben werden. Auf jeden Fall ist darauf zu achten, daß genügend Säure vorhanden ist, um das Kobaltsalz in Lösung zu halten. Das gleiche gilt für die anzuwendende Wassermenge. Um die Kobaltlösung nicht in zu großer Verdünnung zu erhalten, ist es zweckmäßig, die wäßrige kobalthaltige Lösung im Kreis in den Behandlungsraum zurückzuführen und nur im kleinen Teilstrom, der der zugeführten Menge entspricht, abzutrennen.

Die Behandlung erfolgt vorteilhaft bei Temperaturen von 80 bis 160°C, bewährt haben sich insbesondere Temperaturen von 100 bis 130°C. Je nach Grad der Durchmischung ist die Umsetzung bereits nach wenigen Sekunden, vielfach bereits innerhalb von Bruchteilen einer Sekunde beendet. Um eine gute Durchmischung zu gewährleisten, ist es zweckmäßig, das Reaktionsgemisch unter gleichzeitiger Zuführung des Oxidationsmittels, z. B. in feiner Verteilung der wäßrig-sauren Lösung zuzuführen.

Nach der Behandlung trennt man die Flüssigphase z. B. durch Dekantieren in eine organische Phase und eine wäßrige Phase. Aus der organischen Phase erhält man durch fraktionier-

te Destillation nicht umgesetzten Pentensäure-eester, der wieder der Carbonylierung zugeführt werden kann, ferner Valeriansäureester sowie ein Gemisch aus Butandicarbonsäureestern. Das Estergemisch kann zur Herstellung von Diolen oder Polyestern verwendet werden. Der aus dem Estergemisch durch fraktionierte Destillation erhältliche Adipinsäureester eignet sich zur Herstellung von Adipinsäure, AH-Salz, Adipodinitril und Hexandiol-1,6.

Die Kobaltsalze, gegebenenfalls etwas freie Säure enthaltende sowie Alkanole und tertiäre Stickstoffbasen enthaltende wäßrige Phase wird, bevor sie in die Stufe a) als Ausgangslö-sung zur Herstellung von Kobaltcarbonylwasser-stoff zurückgeführt wird, in der Stufe f) mit Lösungsmitteln extrahiert, die mit Wasser nicht mischbar sind. Erfindungsgemäß verwendet man als Extraktionsmittel in der Stufe f) $C_1$- bis $C_5$-Alkan- und/oder Alkencarbonsäure, $C_1$- bis $C_4$-Alkylester. Insbesondere haben sich Ester bewährt, die einen Siedepunkt über 65° C haben. Besondere technische Bedeutung haben $C_4$- bis $C_5$-Alkancarbonsäure. $C_1$- bis $C_4$-Alkyl- und/oder Alkenester erlangt, insbesondere Valeriansä-reester und Pentensäureester. Als technisch bevorzugte Lösung hat es sich herausgestellt, Valeriansäurealkylester und/oder Pentensäurealkylester, die bei dem erfindungsgemäßen Verfahren anfallen, als Extraktionsmittel zu verwenden.

Zur Durchführung der Extraktion eignen sich alle in der Technik eingeführten Verfahrensweisen, wie beispielsweise Gleich- und Gegenstromextraktion und Apparate, wie beispielsweise Pulsationskolonnen, statische Mischer und sogenannte Mixer Settler. In der Regel wird die Extraktion bei der Temperatur durchgeführt, bei der die wäßrige Phase nach Abtrennung der organischen Phase anfällt, z. B. bei Temperaturen von 20 bis 60°C. Je Volumenteil Kobaltsalze enthaltender wäßriger Lösung wird in der Regel 0,05 bis 1 Volumenteil, insbesondere 0,1 bis 0,3 Volumenteile der genannten Alkan- und/oder Alkencarbonsäurealkylester verwendet.

Nach der Extraktion wird die wäßrige Lösung destillativ von Alkanol und Stickstoffbasen befreit und, wie bereits aufgeführt, wieder zur Herstellung von Katalysatorlösung in der Stufe a) verwendet. Die bei der Extraktion anfallende organische Phase wird vorteilhaft dem Reaktionsgemisch in Stufe e) nach der oxidativen Behandlung zugeführt. Hierdurch wird die Phasentrennung erleichtert und zudem extrahierter Butandicarbonsäureester zurückgeführt, so daß eine gesonderte Aufarbeitung nicht erforderlich ist. Der bei der Aufarbeitung der organischen Phase anfallende Alkan- oder Alkensäurealkylester wird dann wieder für die Extraktion verwendet.

Vorteilhaft arbeitet man indem man Valeriansäureester im Gemisch mit Pentensäureester als niedrigsiedende Aceotrope mit Wasser und/oder Aceotrope mit Wasser und Pyridin aus der organischen Phase nach der Phasentrennung in Stufe e) abdestilliert und als zweiphasiges Gemisch für die Extraktion verwendet. Hierzu läßt man der Extraktionsmittelkolonne zusätzlich zur organischen Phase Wasser und Pyridin in einer Menge zulaufen, die der Zusammensetzung der Aceotrope und der gewünschten Menge Extraktionsmittel angepaßt ist. Das der wäßrigen Phase der Extraktion solchermaßen zusätzlich zugeführte Pyridin-Wasser-Gemisch fällt bei der Aufarbeitung der wäßrigen Phase nach Abtrennung des Alkanols wieder an und kann dem Zulauf der Extraktionsmittelkolonne in Stufe e) zurückgeführt werden. Die Extraktion mittels eines binären und/oder ternären Vale-riansäurealkylester-Aceotrops und/oder Penten-säurealkylester-Aceotrops unterscheidet sich im Ergebnis nicht von den Ergebnissen der Extraktion mit reinen Valeriansäurealkylestern oder Pentensäurealkylestern.

Das Verfahren nach der Erfindung sei in folgenden Beispielen veranschaulicht.

## Beispiel 1

Ein Hochdruckrohr, das mit 600 ml Aktivkohle (Firma Norit, Körnung 3 bis 5 mm) gefüllt ist, wird stündlich mit 180 ml einer wäßrigen Kobaltacetatlösung, die 2,5 Gew.-% Kobalt$^{2+}$ enthält, beschickt. Die wäßrige Kobaltacetatlösung fällt in der Stufe e) als wäßrige Phase an. Außerdem führt man stündlich 50 Normalliter eines äquimolaren Gemisches aus Kohlenmonoxid und Wasserstoff zu. Hierbei hält man eine Temperatur von 120°C und einen Druck von 300 bar ein. Die im unteren Teil des Rohres abgezogene Lösung enthält 0,65 Gew.-% Kobalt$^{2+}$ und 1,85 Gew.-% Kobalt als Kobaltcarbonylwasserstoff, ferner die entsprechende Menge an Essigsäure. Diese Lösung wird nach dem Entspannen auf 20 bar bei Raumtemperatur intensiv mit 310 ml eines $C_4$-Schnittes, der 43 Gew.-% Butadien (1,57 Mol) enthält, gemischt. Nach der Phasentrennung enthält der $C_4$-Schnitt 3,7 g Kobalt in Form von Kobaltcarbonylverbindungen. Dieser Kobalt ent-haltende $C_4$-Schnitt wird nun einem Hochdruck-gefäß von 1,9 l Inhalt zugeführt und außerdem stündlich 127 ml (1,57 Mol) Pyridin, 127 ml Methanol (3,14 Mol) und 60 Normalliter Kohlenmonoxid zugegeben. Die Carbonylierung verläuft bei einer Temperatur von 130°C und 600 bar. Das im Kopf des Hochdruckgefäßes abgenommene Produkt wird entspannt, wobei gasförmig neben überschüssigem Kohlenmonoxid überschüssige $C_4$-Kohlenwasserstoffe abgetrennt werden. Die-se enthalten praktisch kein Butadien. Unter vermindertem Druck, um den Katalysator zu schonen, werden von dem Austrag stündlich etwa 52 g Methanol und 100 g Pyridin abdestilliert. Im Destillationssumpf hält man eine maximale Temperatur von 65°C ein. Dieser Destillationssumpf, der 3,7 g Kobalt als Carbonylkomplex und 165 g (1,44 Mol) Pentensäureester enthält, wird zusammen mit 117 ml (2,88 Mol) Methanol und 55 Normalliter Kohlenmonoxid, das 2 Vol.-% Wasserstoff enthält, einem weiteren Hochdruckgefäß von 1,7 Liter Inhalt kontinuierlich von unten zugeführt. Die Carbonylierung wird bei einer Temperatur von 170°C und unter einem Druck von 150 bar durchgeführt. Der Reaktionsaustrag wird mit 200 ml pro Stunde der in der Extraktionsstufe b) anfallenden wäßrigen Lösung unter Durchleiten von etwa 200 Normallitern Luft bei 100°C in einem mit Raschigringen gefüllten Rohr gut durchmischt.

Nach der Phasentrennung wird die organische Phase zusammen mit 50 ml eines aus der Aufarbeitung der wäßrigen Phase stammenden Pyridin-Wasser-Gemischs (etwa 50% Pyridin) einer Destillationskolonne zugeleitet. Der hauptsächlich aus Pyridin, Valeriansäuremethylester, Pentensäuremethylester und Butandicarbonsäuremethylestern bestehende Sumpfaustrag wird einer fraktionierten Destillation unterworfen. Der zweiphasige Kopfaustrag (100 ml pro Stunde) setzt sich zu etwa 40% aus Valeriansäuremethylester, zu etwa 10% aus Pentensäuremethylester und zu je etwa 25% aus Pyridin und Wasser zusammen und wird direkt in eine Gegenstrompulsationskolonne von 1,5 m Länge und 30 m Durchmesser eingepumpt. Die wäßrige Phase enthält nach der Extraktion keine Butandicarbonsäureester mehr und wird nach gemeinsamer destillativer Abtrennung von Methanol und Pyridin (als Azeotrop mit Wasser) als 2,5%ige Kobaltacetatlösung (22 ml pro Stunde) in Stufe a) zurückgeführt. In einer weiteren Destillation werden stündlich 30 ml Methanol als Kopfprodukt und 90 ml Pyridin-Wasser-Gemisch als Sumpfprodukt erhalten. 50 ml pro Stunde des Pyridin-Wasser-Gemischs werden zur Extraktionsmittelkolonne zurückgefahren, die restlichen 40 ml werden, ebenso wie die organische Phase aus der Extraktion, zum Produktstrom vor der Phasentrennung zurückgefahren.

### Vergleichsbeispiel

Es wird wie in Beispiel 1 verfahren, jedoch mit 50 ml pro Stunde Cyclohexan statt des Valeriansäuremethylester-Pentensäuremethylester-Gemischs extrahiert und das bei der Aufarbeitung der wäßrigen Phase anfallende Pyridin-Wasser-Gemisch vollständig zum Produktstrom vor der Phasentrennung zurückgefahren.

Die wäßrige Phase weist nach der Extraktion noch einen Gehalt von 0,4% Butandicarbonsäuredimethylestern auf. Durch teilweise Verseifung dieser Ester in der Katalysatorbildung in Stufe a) pegelt sich bereits nach 4 Kreisläufen ein Gehalt von 0,6% Butandicarbonsäuremonomethylestern und von etwa 0,8% Butandicarbonsäuren auf. In der Folge fallen schwerlösliche Kobaltpyridinadipate und -methylglutarate aus und führen damit zu Leitungsverlegungen und Katalysatorverlusten.

### Patentansprüche

1. Verfahren zur Herstellung von Butandicarbonsäureestern, bei dem man Butadien oder Butadien enthaltende Kohlenwasserstoffgemische mit Kohlenmonoxid und $C_1$- bis $C_4$-Alkanolen in Gegenwart von tertiären Stickstoffbasen und Kobaltcarbonylen bei 80 bis 150°C unter erhöhtem Druck umsetzt und ohne den Katalysator abzutrennen dann den erhaltenen Pentensäureester mit Kohlenmonoxid und $C_1$- bis $C_4$-Alkanolen bei Temperaturen von 140 bis 200°C unter erhöhtem Druck zu Butandicarbonsäureestern umsetzt, wobei man

a) eine wäßrige Kobaltsalzlösung bei Temperaturen von 50 bis 200°C unter Drücken von 50 bis 500 bar mit überschüssigem Kohlenmonoxid und Wasserstoff in Gegenwart von Aktivkohle, die mit Kobaltcarbonyl beladen ist, behandelt;

b) die erhaltene wäßrige Lösung von Kobaltcarbonylwasserstoff mit Butadien oder Butadien enthaltenden Kohlenwasserstoffgemischen extrahiert und die wäßrige Phase abtrennt;

c) das Kobaltcarbonylhydrid, Kobaltcarbonyl und Butenylkobalttricarbonyl enthaltende Butadien oder Butadien-Kohlenwasserstoffgemisch mit Kohlenmonoxid und $C_1$- bis $C_4$-Alkanolen im Überschuß in Gegenwart von 0,5 bis 2 Mol tertiäre Stickstoffbasen je Mol Butadien mit einem $pK_a$-Wert von 3 bis 11 bei Temperaturen von 80 bis 150°C und unter Drücken von 300 bis 2000 bar umsetzt;

d) aus dem erhaltenen Reaktionsgemisch die darin enthaltenen tertiären Stickstoffbasen bis auf 0,1 bis 0,3 Mol je Mol Pentensäureester sowie überschüssige Kohlenwasserstoffe abtrennt, den im Reaktionsgemisch verbleibenden Pentenester mit Kohlenmonoxid und $C_1$- bis $C_4$-Alkanolen im Überschuß bei Temperaturen von 140 bis 200°C und unter Drücken von 100 bis 400 bar in Gegenwart der im Reaktionsgemisch vorhandenen Mengen an Kobaltcarbonyl und tertiären Stickstoffbasen umsetzt;

e) das Kobaltkatalysator, Butandicarbonsäureester, Alkanole, tertiäre Stickstoffbasen, Pentensäureester und Nebenprodukte enthaltende Reaktionsgemisch mit Oxidationsmitteln in Gegenwart der wäßrig-sauren Lösung, die in Stufe b) abgetrennt wurde, behandelt und das Gemisch in eine organische Phase, aus der durch Destillation Butandicarbonsäureester gewonnen werden, und eine wäßrige Phase trennt und

f) die wäßrige Phase mit Lösungsmittel extrahiert, die mit Wasser nicht mischbar sind, die Phasen trennt und die so erhaltene wäßrige Phase nach Abdestillieren von Alkanolen und tertiären Stickstoffbasen in die Stufe a) zurückführt,

dadurch gekennzeichnet, daß man als Extraktionsmittel in der Stufe f) C₁- bis C₅-Alkan- und/oder Alkencarbonsäure C₁- bis C₄-Alkylester verwendet.

2. Verfahren nach Anspruch 1, dadurch gekennzeichnet, daß man C₄- bis C₅-Alkan- und/oder Alkencarbonsäure C₁- bis C₄-Alkylester als Extraktionsmittel verwendet.

3. Verfahren nach den Ansprüchen 1 und 2, dadurch gekennzeichnet, daß man den als Nebenprodukt anfallenden Valeriansäurealkylester und/oder Pentensäurealkylester als Extraktionsmittel verwendet.

4. Verfahren nach den Ansprüchen 1 bis 3, dadurch gekennzeichnet, daß man ein binäres Azeotrop von Valeriansäurealkylester mit Wasser oder ein ternäres Azeotrop aus Valeriansäureester mit Wasser und Pyridin als Extraktionsmittel verwendet.

5. Verfahren nach den Ansprüchen 1 bis 4, dadurch gekennzeichnet, daß man ein binäres Aceotrop von Pentensäuremethylester mit Wasser oder ein ternäres Aceotrop aus Pentensäuremethylester mit Wasser und Pyridin oder ein Gemisch der binären und/oder ternären Aceotrope von Valeriansäuremethylester und Pentensäuremethylester mit Wasser bzw. Wasser und Pyridin als Extraktionsmittel verwendet.

6. Verfahren nach den Ansprüchen 1 bis 5, dadurch gekennzeichnet, daß man die in der Stufe f) nach der Extraktion erhaltene organische Phase in die Stufe e) nach der oxidativen Behandlung zurückführt.

## Claims

1. A process for the preparation of butanedicarboxylic acid esters, wherein butadiene or a butadiene containing hydrocarbon mixture is reacted with carbon monoxide and a $C_1 - C_4$-alkanol in the presence of a tertiary nitrogen base and cobalt carbonyl at from 80 to 150°C under superatmospheric pressure, and the resulting pentenoic acid ester is further reacted with carbon monoxide and a $C_1 - C_4$-alkanol at from 140 to 200°C under superatmospheric pressure, to give butanedicarboxylic acid esters,

a) an aqueous cobalt salt solution being treated, at from 50 to 200°C and under a pressure of from 50 to 500 bar, with excess carbon monoxide and hydrogen in the presence of active charcoal which has been laden with cobalt carbonyl,

b) the resulting aqueous solution of cobalt carbonyl hydride being extracted with butadiene or a butadiene-containing hydrocarbon mixture, and the aqueous phase separated off,

c) the butadiene, or butadiene/hydrocarbon mixture, containing cobalt carbonyl hydride, cobalt carbonyl and butenyl-cobalt tricarbonyl, being reacted with carbon monoxide and excess $C_1 - C_4$-alkanol in the presence of from 0.5 to 2 moles, per mole of butadiene, of a tertiary nitrogen base having a $pK_a$ of from 3 to 11, at from 80 to 150°C under a pressure of from 300 to 2000 bar,

d) the resulting reaction mixture being freed from the tertiary notrogen base contained therein, down to a content of from 0.1 to 0.3 mole per mole of pentenoic acid ester, and from excess hydrocarbons, and the pentenoic acid ester remaining in the reaction mixture being reacted with carbon monoxide and excess $C_1 - C_4$-alkanol at from 140 to 200°C and under a pressure of from 100 to 400 bar in the presence of the amounts of cobalt carbonyl and tertiary nitrogen base contained in the reaction mixture,

e) the reaction mixture containing cobalt catalyst, butanedicarboxylic acid esters, alkanols, tertiary nitrogen base, pentenoic acid esters and by-products being treated with an oxidizing agent in the presence of the aqueous acid solution which has been separated off in stage b), and the mixture being separated into an organic phase, from which butanedicarboxylic acid esters are isolated by distillation, and an aqueous phase, and

f) the aqueous phase being extracted with a solvent which is immiscible with water, the phases being separated, and the aqueous phase thus obtained being freed from alkanols and tertiary nitrogen base by distilling these off, and recycled to stage a),

characterized in that $C_1 - C_4$-alkyl esters of $C_1 - C_5$-alkanecarboxylic acids and/or alkenecarboxylic acids are used as the extractant in stage f).

2. A process as claimed in claim 1, characterized in that $C_1 - C_4$-alkyl esters of $C_4 - C_5$-alkanecarboxylic acids and/or $C_4 - C_5$-alkenecarboxylic acids are used as the extractant.

3. A process as claimed in claims 1 and 2, characterized in that the alkyl valerate and/or alkyl pentenoate obtained as a by-product is used as the extractant.

4. A process as claimed in claims 1 to 3, characterized in that a binary azeotrope of alkyl valerate with water, or a ternary azeotrope of alkyl valerate with water and pyridine is used as the extractant.

5. A process as claimed in claims 1 to 4, characterized in that a binary azeotrope of methyl pentenoate with water, or a ternary azeotrope of methyl pentenoate with water and pyridine, or a mixture of the binary and/or ternary azeotrope of methyl valerate and methyl pentenoate with water, or with water and pyridine, is used as the extractant.

6. A process as claimed in claims 1 to 5, characterized in that the organic phase obtained after the extraction in stage f) is recycled to stage e) after the oxidative treatment.

**Revendications**

1. Procédé de préparation d'esters d'acide butanedicarboxylique par réaction du butadiène ou de mélanges d'hydrocarbures contenant du butadiène avec l'oxyde de carbone et des alcanols en $C_1$ à $C_4$ en présence de bases azotées tertiaires et de cobalt-carbonyles, sous pression accrue et à des températures de 80 à 150°C, suivie de la transformation de l'ester d'acide penténique formé, sans en séparer le catalyseur, par réaction avec l'oxyde de carbone et les alcanols en $C_1$ à $C_4$, sous pression accrue entre 140 et 200°C, en esters d'acide butane-dicarboxylique, dans lequel:

a) on traite une solution aqueuse d'un sel de cobalt à des températures de 50 à 200°C et sous des pressions de 50 à 500 bars, en présence de charbon activé chargé de cobalt-carbonyle, avec de l'hydrogène et de l'oxyde de carbone en excès;

b) on extrait la solution aqueuse de cobalt-carbonyle-hydrogène obtenue avec du butadiène ou un mélange d'hydrocarbures contenant du butadiène et on sépare la phase aqueuse;

c) on fait réagir le butadiène ou le mélange d'hydrocarbures au butadiène, contenant de l'hydrure de cobalt-carbonyle, du cobalt-carbonyle et du butényl-cobalt-tricarbonyle, à des températures de 80 à 150°C et sous des pressions de 300 à 2000 bars, avec de l'oxyde de carbone et des alcanols en $C_1$ à $C_4$ en excès en présence de 0,5 à 2 moles par mole de butadiène de bases azotées tertiaires avec un $pK_a$ de 3 à 11;

d) on sépare du mélange réactionnel obtenu les hydrocarbures en excès et on en réduit la teneur en bases azotées tertiaires entre 0,1 et 0,3 mole par mole d'ester d'acide penténique, puis on fait réagir l'ester penténique contenu dans le mélange réactionnel entre 140 et 200°C sous 100 à 400 bars avec l'oxyde de carbone et les alcanols en $C_1$ à $C_4$ en excès en présence du cobalt-carbonyle et des bases azotées tertiaires résiduels;

e) on soumet le mélange réactionnel contenant le catalyseur au cobalt, des esters d'acide butane-dicarboxylique, des alcanols, des bases azotées tertiaires, des esters d'acide penténique et des produits secondaires, en présence de la solution aqueuse acide séparée dans le stade b), à un traitement avec des agents d'oxydation, puis on sépare le mélange en une phase organique, de laquelle on obtient par distillation les esters d'acide butane-dicarboxylique, et une phase aqueuse,

f) cette phase aqueuse étant extraite à l'aide de solvants non miscibles à l'eau et, après séparation des phases, la phase aqueuse soumise à une distillation pour en éliminer les alcanols et les bases azotées tertiaires, puis recyclée dans le stade a),

caractérisé en ce que l'extraction dans le stade f) est réalisée à l'aide d'esters d'alcoyle en $C_1$ à $C_4$ d'acides alcane- et(ou) alcène-carboxyliques en $C_1$ à $C_5$.

2. Procédé suivant la revendication 1, caractérisé en ce que l'on emploie des esters d'alcoyle en $C_1$ à $C_4$ d'acides alcane- et(ou) alcène-carboxyliques en $C_4$ ou $C_5$.

3. Procédé suivant l'une des revendications 1 et 2, caractérisé en ce que l'extraction est réalisée à l'aide des esters d'alcoyle de l'acide valérique et(ou) de l'acide penténique formés comme sous-produits.

4. Procédé suivant l'une des revendications 1 à 3, caractérisé en ce que l'on emploie comme agent d'extraction, soit un mélange azéotrope binaire valérate d'alcoyle-eau, soit un mélange azéotrope ternaire valérate d'alcoyle-eau-pyridine.

5. Procédé suivant l'une des revendications 1 à 4, caractérisé en ce que l'on emploie comme agent d'extraction un mélange azéotrope binaire ester méthylique d'acide penténique-eau ou un mélange azéotrope ternaire ester méthylique d'acide penténique-eau-pyridine ou un mélange des azéotropes binaires et(ou) ternaires d'ester méthylique d'acide valérique ou d'acide penténique et d'eau ou d'eau et de pyridine.

6. Procédé suivant l'une des revendications 1 à 5, caractérisé en ce que la phase organique obtenue dans le stade f) après l'extraction est recyclée dans le stade e) après le traitement avec les agents oxydants.